# EUROPEAN PATENT APPLICATION

(11) **EP 4 509 190 A1**
(43) Date of publication of application: **19.02.2025**
(21) Application number: 23788163.6
(22) Date of filing: 28.03.2023
(51) Int. Cl.: A61Q 1/12, A61K 8/25, A61K 8/31, A61K 8/37, A61K 8/891, A61K 8/894, A61K 8/898

(54) **GELLING AGENT, METHOD FOR PRODUCING GELLING AGENT AND COSMETIC PREPARATION**

(30) Priority: 13.04.2022 JP 2022066212
(71) Applicant: SHIN-ETSU CHEMICAL CO., LTD., Tokyo 1000005 (JP)
(72) Inventor: IMAI Taro, Annaka-shi, Gunma 379-0224 (JP)
(74) Representative: Sonnenhauser, Thomas Martin
(86) International application number: PCT/JP2023/012537
(87) International publication number: WO 2023/199743

(57) **Abstract**

The present invention is a gelling agent including a water-swellable clay mineral in which ammonium group-modified organopolysiloxane represented by the following average composition formula (1) is incorporated in an interlayer space.

(R¹₃SiO_{1/2})ₐ₁ (R²ₙR¹₃₋ₙSiO_{1/2})ₐ₂(R¹₂SiO_{2/2})_{b}(R¹SiO_{3/2})_{c}(SiO_{4/2})_{d} (1)

Thus, the present invention particularly thickens silicone oil to provide a gelled product that is stable over time.

## Description

### TECHNICAL FIELD

The present invention relates to a gelling agent, a method for producing a gelling agent, and a cosmetic.

### BACKGROUND ART

Silicone oil has light spreadability as well as excellent smoothness and water repellency, and a cosmetic containing silicone oil can give light texture. However, compatibility with oils other than silicone oil is limited, and to stabilize a cosmetic containing them, it is necessary to solidify or thicken silicone oil.

As a method for thickening silicone oil, methods using crosslinked organopolysiloxane (Patent Document 1) or using a gelling agent with an aromatic group coupled to a siloxane chain via an amide bond (Patent Document 2) have been reported. However, if a large amount of such gelling agent is added, sticky feeling or heavy texture may be imparted.

As other methods for thickening silicone oil, a method that blends a clay mineral having a silicone compound incorporated in an interlayer space has been reported. However, ammonium salt-containing organopolysiloxane for use is limited to a dual-end modified or side-chain modified type, and a thickening effect and stability over time thereof are insufficient (Patent Document 3).

### CITATION LIST

### PATENT LITERATURE

Patent Document 1: JP 2016-169324 A
Patent Document 2: WO 2018/163989 A1
Patent Document 3: JP S63-72779 A

### SUMMARY OF INVENTION

### TECHNICAL PROBLEM

The present invention has been made in view of the above circumstances and has an object to provide: a gelling agent excellent in particularly thickening silicone oil to yield a gelled product that is stable over time; a method for producing the gelling agent; and a cosmetic containing the gelling agent.

### SOLUTION TO PROBLEM

The present inventor has made an intensive investigation to achieve the above object, and consequently found that a water-swellable clay mineral having single-end type ammonium salt-modified organopolysiloxane incorporated in an interlayer space exhibits an excellent thickening property as a gelling agent for silicone oil. The present inventor has also obtained knowledge that a cosmetic containing the gelling agent is excellent in use feeling and usability as well as stability over time, and made the present invention.

To achieve the above object, the present invention provides:
a gelling agent including a water-swellable clay mineral in which ammonium group-modified organopolysiloxane represented by the following average composition formula (1) is incorporated in an interlayer space,

   (R¹₃SiO_{1/2})ₐ₁(R²ₙR¹₃₋ₙSiO_{1/2})ₐ₂(R¹₂SiO_{2/2})_{b}(R¹SiO_{3/2})_{c}(SiO_{4/2})_{d} (1)
in the formula (1), R¹ independently represents a group selected from an alkyl group having 1 to 10 carbon atoms, an aryl group having 6 to 10 carbon atoms, and an arylene group having 7 to 10 carbon atoms,
R² represents an ammonium group represented by the following formula (2),
"a1" satisfies 0 < a1 ≤ 20, "a2" satisfies 0 < a2 ≤ 12, "b" satisfies 0 ≤ b ≤ 150, "c" satisfies 0 ≤ c ≤ 10, and "d" satisfies 0 ≤ d ≤ 5, provided that a1 + a2 + b + c + d is 2 to 170; and "n" represents an integer of 1 to 3, 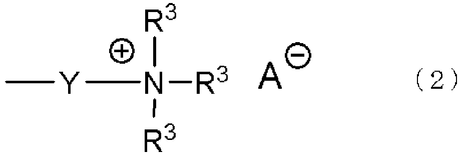
in the formula (2), R³ independently represents a hydrogen atom or a group selected from an alkyl group having 1 to 20 carbon atoms, an aryl group having 6 to 10 carbon atoms, and an arylene group having 7 to 10 carbon atoms,
Y represents a group represented by any of following formulae (3), and
A⁻ represents an anion that is a counterion to an ammonium ion, 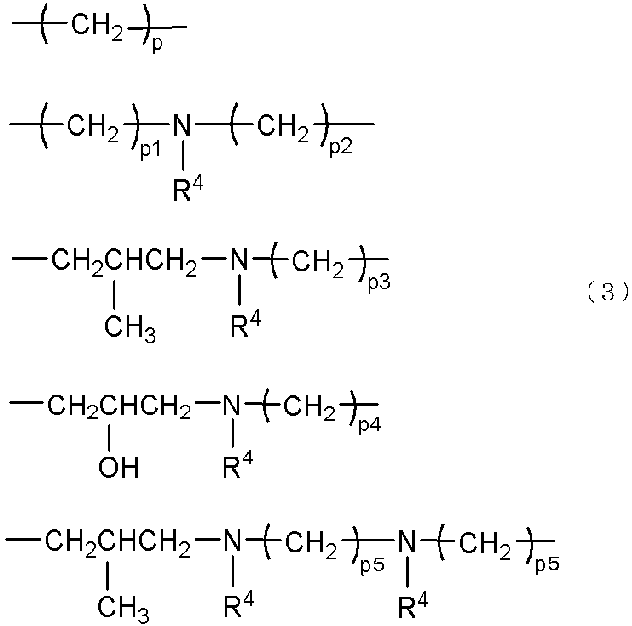
wherein "p" represents an integer of 1 ≤ p ≤ 10; "p1" satisfies 1 ≤ p1 ≤ 10 and "p2" represents an integer of 1 ≤ p2 ≤ 10, provided 1 ≤ p1 + p2 ≤ 15; "p3" represents an integer of 1 ≤ p3 ≤ 10; "p4" represents an integer of 1 ≤ p4 ≤ 12; and "p5" represents an integer of 1 ≤ p5 ≤ 10, and
R⁴ represents a hydrogen atom or a group selected from an alkyl group having 1 to 20 carbon atoms, an aryl group having 6 to 10 carbon atoms, and an arylene group having 7 to 10 carbon atoms.

Such gelling agent can provide a gelling agent excellent in particularly thickening silicone oil to yield a gelled product that is stable over time.

Furthermore, the anion represented as A⁻ in the formula (2) is preferably an anion selected from a hydroxide ion, a chloride ion, a bromide ion, a nitrate ion, a hydrogen sulfate ion, a hydrogen carbonate ion, and an acetate ion.

Such gelling agent can be more excellent in a thickening property and stability over time.

Furthermore, the ammonium group-modified organopolysiloxane is preferably represented by the following formula (4), 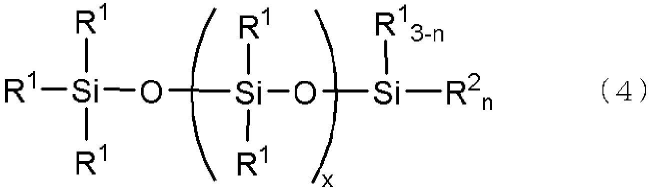 in the formula (4), R¹, R², and "n" are same as above; and "x" satisfies 0 ≤ x ≤ 100.

Such gelling agent can be particularly excellent in a thickening property and stability over time.

The water-swellable clay mineral is preferably one or more water-swellable clay minerals selected from montmorillonite, saponite, smectite, hectorite, sodium silicic mica, sodium tainiolite, or lithium tainiolite.

The suitable water-swellable clay mineral is as described above.

Furthermore, the present invention provides a cosmetic including a gelling agent.

Such cosmetic can provide a cosmetic that has excellent adhesion, provides a natural finish, and also has excellent stability over time.

Furthermore, the cosmetic preferably further includes oil in a liquid state at 25°C.

Such cosmetic can provide a cosmetic that has excellent adhesion, provides a natural finish, and also has more excellent stability over time.

Furthermore, the oil preferably includes silicone oil.

Particularly, silicone oil is suitable as the oil.

Furthermore, the present invention provides a method for producing a gelling agent, including a step of:
treating a water-swellable clay mineral with ammonium group-modified organopolysiloxane represented by the following average composition formula (1),

   (R¹₃SiO_{1/2})ₐ₁(R²ₙR¹₃₋ₙSiO_{1/2})ₐ₂(R¹₂SiO_{2/2})_{b}(R¹SiO_{3/2})_{c}(SiO_{4/2})_{d} (1)
in the formula (1), R¹ independently represents a group selected from an alkyl group having 1 to 10 carbon atoms, an aryl group having 6 to 10 carbon atoms, and an arylene group having 7 to 10 carbon atoms,
R² represents an ammonium group represented by the following formula (2),
"a1" satisfies 0 < a1 ≤ 20, "a2" satisfies 0 < a2 ≤ 12, "b" satisfies 0 ≤ b ≤ 150, "c" satisfies 0 ≤ c ≤ 10, and "d" satisfies 0≤d≤5, provided that a1 + a2 + b + c + d is 2 to 170; and "n" represents an integer of 1 to 3, 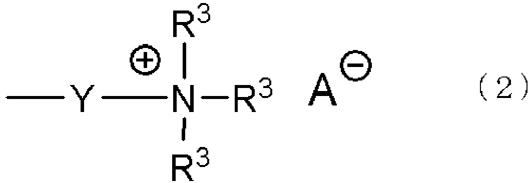
in the formula (2), R³ independently represents a hydrogen atom or a group selected from an alkyl group having 1 to 20 carbon atoms, an aryl group having 6 to 10 carbon atoms, and an arylene group having 7 to 10 carbon atoms,
Y represents a group represented by any of following formulae (3), and
A⁻ represents an anion that is a counterion to an ammonium ion, 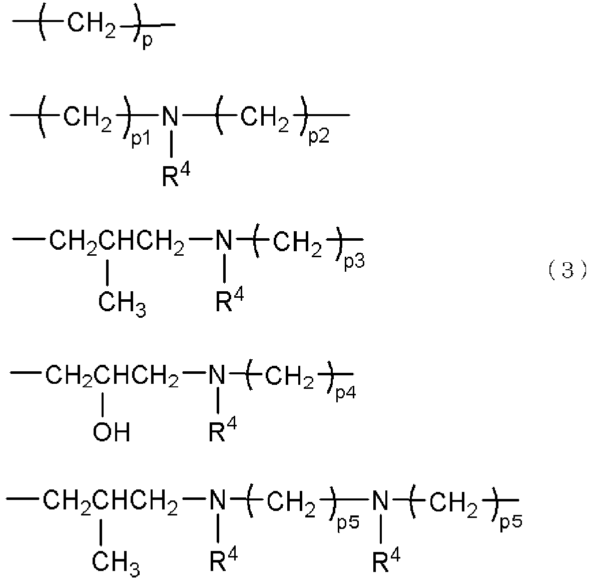
wherein "p" represents an integer of 1 ≤ p ≤ 10; "p1" satisfies 1 ≤ p1 ≤ 10 and "p2" represents an integer of 1≤p2≤10, provided 1 ≤ p1 + p2 ≤ 15; "p3" represents an integer of 1≤p3≤10; "p4" represents an integer of 1 ≤ p4 ≤ 12; and "p5" represents an integer of 1≤p5≤10, and
R⁴ represents a hydrogen atom or a group selected from an alkyl group having 1 to 20 carbon atoms, an aryl group having 6 to 10 carbon atoms, and an arylene group having 7 to 10 carbon atoms.

Such method for producing a gelling agent can yield a gelling agent that is excellent in a thickening property and stability over time.

Furthermore, the anion represented as A⁻ in the formula (2) is preferably an anion selected from a hydroxide ion, a chloride ion, a bromide ion, a nitrate ion, a hydrogen sulfate ion, a hydrogen carbonate ion, and an acetate ion.

Such method for producing a gelling agent can yield a gelling agent that is more excellent in a thickening property and stability over time.

Furthermore, a compound represented by the following formula (4) is preferably used as the ammonium group-modified organopolysiloxane, 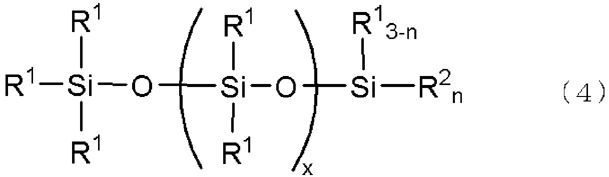 in the formula (4), R¹ ,R², and "n" are same as above; and "x" satisfies 0 ≤ x ≤ 100.

Such method for producing a gelling agent can yield a gelling agent that is particularly excellent in a thickening property and stability over time.

Furthermore, one or more water-swellable clay minerals selected from montmorillonite, saponite, smectite, hectorite, sodium silicic mica, sodium tainiolite, or lithium tainiolite are preferably used as the water-swellable clay mineral.

The suitable water-swellable clay mineral is as described above.

### ADVANTAGEOUS EFFECTS OF INVENTION

The gelling agent of the present invention can thicken oil in a liquid state at 25°C, particularly silicone oil, and yield a gelled product that is stable over time. Furthermore, a cosmetic containing the gelling agent has no sticky feeling or heavy texture after application, and can be lightly spread and expanded with silky smooth feeling and provide smooth texture.

### DESCRIPTION OF EMBODIMENTS

As described above, it has been demanded to develop a gelling agent excellent in particularly thickening silicone oil to yield a gelled product that is stable over time.

As a result of an intensive investigation on the above problems, the present inventor found that the gelling agent of the present invention can thicken oil in a liquid state at 25°C, particularly silicone oil, and yield a gelled product that is stable over time, and a cosmetic containing the gelled product has no sticky feeling or heavy texture after application and can be lightly spread and expanded with silky smooth feeling and provide smooth texture, and completed the present invention.

Thus, the present invention is a gelling agent including a water-swellable clay mineral in which ammonium group-modified organopolysiloxane represented by the following average composition formula (1) is incorporated in an interlayer space,

(R¹₃SiO_{1/2})ₐ₁(R²ₙR¹₃₋ₙSiO_{1/2})ₐ₂(R¹₂SiO_{2/2})_{b}(R¹SiO_{3/2})_{c}(SiO_{4/2})_{d} (1)

in the formula (1), R¹ independently represents a group selected from an alkyl group having 1 to 10 carbon atoms, an aryl group having 6 to 10 carbon atoms, and an arylene group having 7 to 10 carbon atoms,
R² represents an ammonium group represented by the following formula (2),
"a1" satisfies 0 < a1 ≤ 20, "a2" satisfies 0 < a2 ≤ 12, "b" satisfies 0 ≤ b ≤ 150, "c" satisfies 0 ≤ c ≤ 10, and "d" satisfies 0 ≤ d ≤ 5, provided that a1 + a2 + b + c + d is 2 to 170; and "n" represents an integer of 1 to 3, 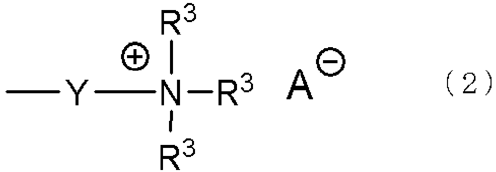
in the formula (2), R³ independently represents a hydrogen atom or a group selected from an alkyl group having 1 to 20 carbon atoms, an aryl group having 6 to 10 carbon atoms, and an arylene group having 7 to 10 carbon atoms,
Y represents a group represented by any of following formulae (3), and
A⁻ represents an anion that is a counterion to an ammonium ion, 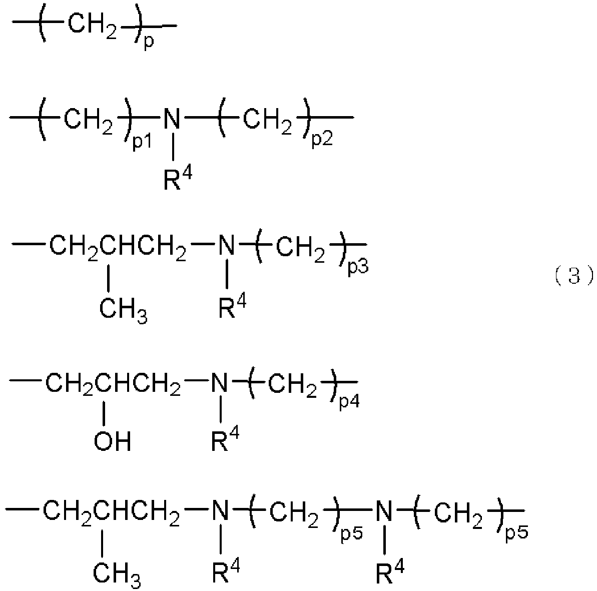
wherein "p" represents an integer of 1 ≤ p ≤ 10; "p1" satisfies 1 ≤ p1 ≤ 10 and "p2" represents an integer of 1 ≤ p2 ≤ 10, provided 1 ≤ p1 + p2 ≤ 15; "p3" represents an integer of 1 ≤ p3 ≤ 10; "p4" represents an integer of 1 ≤ p4 ≤ 12; and "p5" represents an integer of 1 ≤ p5 ≤ 10, and
R⁴ represents a hydrogen atom or a group selected from an alkyl group having 1 to 20 carbon atoms, an aryl group having 6 to 10 carbon atoms, and an arylene group having 7 to 10 carbon atoms.

Hereinafter, the present invention will be described in detail. However, the present invention is not limited thereto.

### (A) Gelling Agent

(A) the gelling agent of the present invention is a gelling agent including a water-swellable clay mineral in which ammonium group-modified organopolysiloxane represented by the following average composition formula (1) is incorporated in an interlayer space, and can be used alone or in combination of two or more.

(R¹₃SiO_{1/2})ₐ₁(R²ₙR¹₃₋ₙSiO_{1/2})ₐ₂(R¹₂SiO_{2/2})_{b}(R¹SiO_{3/2})_{c}(SiO_{4/2})_{d} (1)

In the formula (1), R¹ independently represents a group selected from an alkyl group having 1 to 10 carbon atoms, an aryl group having 6 to 10 carbon atoms, and an arylene group having 7 to 10 carbon atoms,
R² represents an ammonium group represented by the following formula (2),
"a1" satisfies 0 < a1 ≤ 20, "a2" satisfies 0 < a2 ≤ 12, "b" satisfies 0 ≤ b ≤ 150, "c" satisfies 0 ≤ c ≤ 10, and "d" satisfies 0 ≤ d ≤ 5, provided that a1 + a2 + b + c + d is 2 to 170; and "n" represents an integer of 1 to 3, 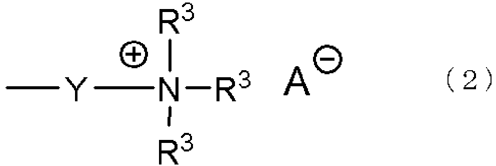
in the formula (2), R³ independently represents a hydrogen atom or a group selected from an alkyl group having 1 to 20 carbon atoms, an aryl group having 6 to 10 carbon atoms, and an arylene group having 7 to 10 carbon atoms,
Y represents a group represented by any of following formulae (3), and
A⁻ represents an anion that is a counterion to an ammonium ion, 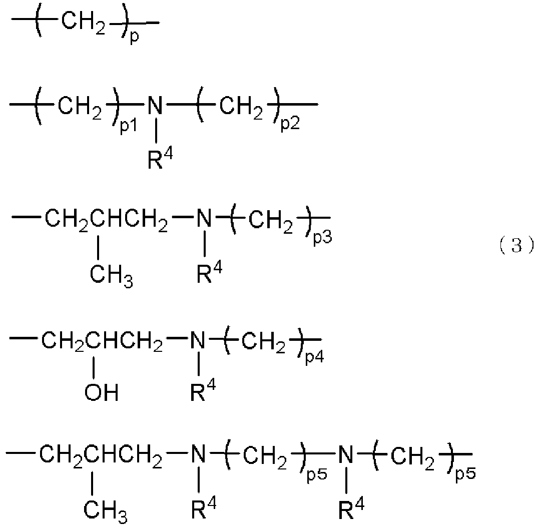
wherein "p" represents an integer of 1 ≤ p ≤ 10; "p1" satisfies 1 ≤ p1 ≤ 10 and "p2" represents an integer of 1 ≤ p2 ≤ 10, provided 1 ≤ p1 + p2 ≤ 15; "p3" represents an integer of 1 ≤ p3 ≤ 10; "p4" represents an integer of 1 ≤ p4 ≤ 12; and "p5" represents an integer of 1 ≤ p5 ≤ 10, and
R⁴ represents a hydrogen atom or a group selected from an alkyl group having 1 to 20 carbon atoms, an aryl group having 6 to 10 carbon atoms, and an arylene group having 7 to 10 carbon atoms.

### Ammonium Group-Modified Organopolysiloxane

The ammonium group-modified organopolysiloxane of the present invention is organopolysiloxane having, in one molecule, one or more quaternary ammonium cations that can be exchanged with an exchangeable cation present in the interlayer space of the clay mineral.

The ammonium group-modified organopolysiloxane of the present invention is represented by the following average composition formula (1), and can be used alone or in combination of two or more.

(R¹₃SiO_{1/2})ₐ₁(R²ₙR¹₃₋ₙSiO_{1/2})ₐ₂(R¹₂SiO_{2/2})_{b}(R¹SiO_{3/2})_{c}(SiO_{4/2})_{d} (1)

R¹ independently represents a group selected from an alkyl group having 1 to 10 carbon atoms, an aryl group having 6 to 10 carbon atoms, and an arylene group having 7 to 10 carbon atoms. Specific examples thereof include: alkyl groups such as methyl group, ethyl group, propyl group, butyl group, and pentyl group; cycloalkyl groups such as cyclopentyl group; and aryl groups such as phenyl group and tolyl group. R¹ is preferably a methyl group or phenyl group, more preferably a methyl group.
R² represents an ammonium group represented by the following general formula (2). 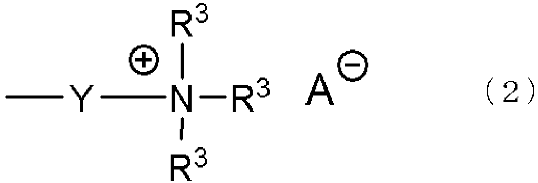
"a1" satisfies 0 < a1 ≤ 20, preferably 0 < a1 ≤ 5. "a2" satisfies 0 < a2 ≤ 12, preferably 1 ≤ a2 ≤ 3. "b" satisfies 0 ≤ b ≤ 150, preferably 0 ≤ b ≤ 100. "c" satisfies 0 ≤ c ≤ 10, preferably 0 ≤ c ≤ 1. "d" satisfies 0≤d≤5, preferably 0≤d≤1. Note that a1 + a2 + b + c + d is 2 to 170, preferably 2 to 110. "n" represents an integer of 1 to 3, preferably 1.
R³ independently represents a hydrogen atom or a group selected from an alkyl group having 1 to 20 carbon atoms, an aryl group having 6 to 10 carbon atoms, and an arylene group having 7 to 10 carbon atoms. Specific examples thereof include: alkyl groups such as methyl group, ethyl group, propyl group, butyl group, pentyl group, lauryl group, stearyl group, and mycyril group; cycloalkyl groups such as cyclopentyl group; and aryl groups such as phenyl group and tolyl group. R³ is preferably a hydrogen atom, methyl group, ethyl group, propyl group, butyl group, pentyl group, lauryl group, stearyl group, or mycyril group; more preferably a hydrogen atom, methyl group, lauryl group, or stearyl group; and further preferably a hydrogen atom or methyl group.
Y represents a group represented by any of following formulae (3), 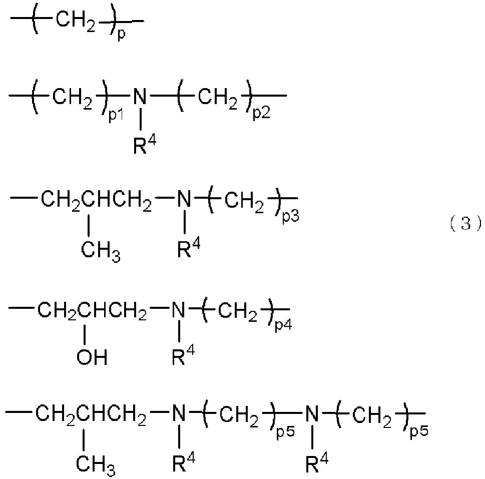
wherein "p" represents an integer of 1 ≤ p ≤ 10; "p1" satisfies 1 ≤ p1 ≤ 10 and "p2" represents an integer of 1 ≤ p2 ≤ 10, provided 1 ≤ p1 + p2 ≤ 15; "p3" represents an integer of 1 ≤ p3 ≤ 10; "p4" represents an integer of 1 ≤ p4 ≤ 12; and "p5" represents an integer of 1 ≤ p5 ≤ 10.
R⁴ represents a hydrogen atom, an alkyl group having 1 to 20 carbon atoms, preferably 1 to 10 carbon atoms, an aryl group having 6 to 10 carbon atoms, or an arylene group having 7 to 10 carbon atoms. Preferred is a hydrogen atom or methyl group, and more preferred is a hydrogen atom.

Specific examples of Y are illustrated as following structures.

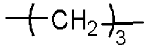

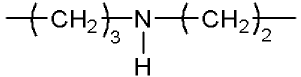

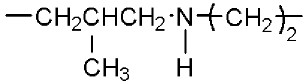

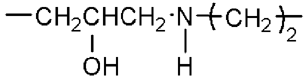

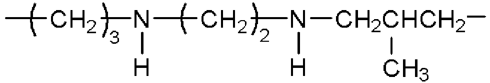

A⁻ represents an anion that is a counterion to an ammonium ion, and specific examples thereof include hydroxide ion, chloride ion, bromide ion, nitrate ion, hydrogen sulfate ion, hydrogen carbonate ion, acetate ion, citrate ion, lactate ion, glutamate ion, aspartate ion, and the like. A⁻ is preferably a chloride ion or acetate ion, more preferably a chloride ion.

As the ammonium group-modified organopolysiloxane, a compound represented by the following formula (4) may be used. The ammonium group-modified organopolysiloxane having such structure is capable of particularly thickening silicone oil to yield a gelled product that is stable over time.

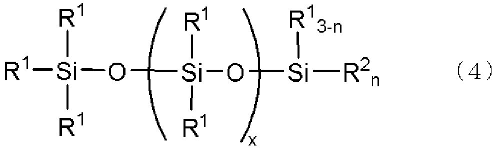

In the formula (4), R¹, R², and "n" are same as above; and "x" satisfies 0 ≤ x ≤ 100.

### Water-Swellable Clay Mineral

Examples of the water-swellable clay mineral used in the present invention include montmorillonite, saponite, smectite, hectorite, sodium silicic mica, sodium tainiolite, lithium tainiolite, and the like. Among these, montmorillonite, saponite, smectite, or hectorite is preferred.

### Method for Producing Gelling Agent

The gelling agent of the present invention is characterized in that treatment is performed to incorporate the ammonium group-modified organopolysiloxane in an interlayer space of the water-swellable clay mineral.

Methods for the above treatment include: a method in which the water-swellable clay mineral is dissolved or dispersed in a low boiling point solvent such as, for example, water, acetone, isopropyl alcohol, or ethanol, the ammonium group-modified organopolysiloxane dissolved in a solvent such as, for example, isopropyl alcohol, ethanol, or cyclopentasiloxane is added thereto and stirred at room temperature or under heating, and thereafter the solvent is distilled off; and a method in which the water-swellable clay mineral and the ammonium group-modified organopolysiloxane are added to a low boiling point solvent such as, for example, water, acetone, isopropyl alcohol, or ethanol, the resultant is stirred at room temperature or under heating, and thereafter the solvent is distilled off. Temperature during heating and stirring (if performed) is preferably 40 to 100°C, more preferably 40 to 60°C.

An amount of the ammonium group-modified organopolysiloxane to be used as a treating agent is preferably 10 to 200 parts by mass, more preferably 50 to 150 parts by mass relative to 100 parts by mass of the water-swellable clay mineral.

In this case, the anion represented as A⁻ in the formula (2) can be an anion selected from a hydroxide ion, a chloride ion, a bromide ion, a nitrate ion, a hydrogen sulfate ion, a hydrogen carbonate ion, and an acetate ion.

Furthermore, a compound represented by the formula (4) can be used as the ammonium group-modified organopolysiloxane.

Furthermore, one or more water-swellable clay minerals selected from montmorillonite, saponite, smectite, hectorite, sodium silicic mica, sodium tainiolite, or lithium tainiolite can be used as the water-swellable clay mineral.

### Cosmetic

The present invention is a cosmetic including the above gelling agent. The cosmetic may further include oil in a liquid state at 25°C. Furthermore, this oil preferably includes silicone oil.

### (B) Oil in Liquid State at 25°C

The cosmetic of the present invention may include (B) oil in a liquid state at 25°C. Kinematic viscosity of the oil at 25°C is 0.5 to 100 mm²/s, more preferably 0.5 to 20 mm²/s. As this oil, those generally added to a cosmetic can be used without particular limitation, and can be used alone or in combination of two or more. Note that in the present description, the kinematic viscosity refers to kinematic viscosity at 25°C measured by a method described in JIS Z 8803:2011 using a Cannon-Feske viscometer.

Examples of (B) the oil in a liquid state at 25°C include hydrocarbon oils, higher fatty acids, natural animal or vegetable oils and fats, semi-synthetic oils and fats, esters, silicone oils, and fluorine oils, which are specifically described as follows.

### - Hydrocarbon Oil

Hydrocarbon oils include straight or branched hydrocarbon oils, which may be volatile or non-volatile hydrocarbon oils. Specific examples thereof include olefin oligomer, isododecane (:labeling name (INCI: Isododecane)), dodecane (:labeling name (INCI: Dodecane)), isohexadecane (:labeling name (INCI: Isohexadecane)), undecane (:labeling name (INCI: Undecane)), squalane (:labeling name (INCI: Squalane)), squalene (:labeling name (INCI: Squalene)), mineral oil (:labeling name (INCI: Mineral Oil)), liquid isoparaffin, polyisobutylene (:labeling name), hydrogenated polyisobutene (:labeling name (INCI: Hydrogenated Polyisobutene)), (C13-15) alkane (:labeling name (INCI: C13-15 Alkane)), and the like.

### - Higher Fatty Acid

Higher fatty acids include oleic acid (:labeling name (INCI: Oleic Acid)), linoleic acid (:labeling name (INCI: Linoleic Acid)), linolenic acid (:labeling name (INCI: Linolenic Acid)), arachidonic acid (:labeling name (INCI: Arachidonic Acid)), eicosapentaenoic acid (EPA)(:labeling name (INCI: Eicosapentaenoic Acid)), docosahexaenoic acid (DHA)(:labeling name (INCI: Docosahexaenoic Acid)), isostearic acid (:labeling name (INCI: Isostearic Acid)), hydroxystearic acid (:labeling name (INCI: Hydroxystearic Acid)), and the like.

### - Natural Animal or Vegetable Oils and Fats and Semi-Synthetic Oils and Fats

Natural animal or vegetable oils and fats and semi-synthetic oils and fats include avocado oil (:labeling name (INCI: Persea Gratissima (Avocado) Oil)), linseed oil (:labeling name (INCI: Linum Usitatissimum (Linseed) Seed Oil)), almond oil (:labeling name (INCI: Prunus Amygdalus Dulcis (Sweet Almond) Oil)), olive oil (:labeling name (INCI: Olea Europaea (Olive) Fruit Oil)), torreya californica oil (:labeling name (INCI: Torreya Californica (California Nutmeg) Oil)), citronella oil (:labeling name (INCI: Cymbopogon Nardus (Citronella) Oil)), shark liver oil (:labeling name (INCI: Shark Liver Oil)), cod liver oil (:labeling name (INCI: Cod Liver Oil)), fish liver oil (:labeling name (INCI: Fish Liver Oil)), apricot kernel oil (:labeling name (INCI: Kyounin Yu)), sesame oil (:labeling name (INCI: Sesamum Indicum (Sesame) Seed Oil)), rice germ oil (:labeling name (INCI: Oryza Sativa (Rice) Germ Oil)), rice bran oil (:labeling name (INCI: Oryza Sativa (Rice) Bran Oil)), camellia kissi seed oil (:labeling name (INCI: Camellia Kissi Seed Oil)), safflower oil (:labeling name (INCI: Carthamus Tinctorius (Safflower) Seed Oil)), turtle oil (:labeling name (INCI: Turtle Oil)), camellia oil (:labeling name (INCI: Camellia Japonica Seed Oil)), evening primrose oil (:labeling name (INCI: Oenothera Biennis (Evening Primrose) Oil)), corn germ oil (:labeling name (INCI: Zea Mays (Corn) Germ Oil)), rapeseed oil (:labeling name (INCI:RAPE SHUSHI YU)), wheat germ oil (:labeling name (INCI: Triticum Vulgare (Wheat) Germ Oil)), persic oil (:labeling name (INCI:)), palm oil (:labeling name (INCI: Elaeis Guineensis (Palm) Oil)), palm kernel oil (:labeling name (INCI: Elaeis Guineensis (Palm) Kernel Oil)), castor oil (:labeling name (INCI: Ricinus Communis (Castor) Seed Oil)), hydrogenated castor oil (:labeling name (INCI: Hydrogenated Castor Oil)), sunflower seed oil (:labeling name (INCI: Helianthus Annuus (Sunflower) Seed Oil)), grape seed oil (:labeling name (INCI: Vitis Vinifera (Grape) Seed Oil)), jojoba oil (:labeling name (INCI: Simmondsia Chinensis (Jojoba) Seed Oil)), macadamia nut oil (:labeling name (INCI: Macadamia Ternifolia Seed Oil)), mink oil (:labeling name (INCI: Mink Oil)), meadowfoam seed oil (:labeling name (INCI: Limnanthes Alba (Meadowfoam) Seed Oil)), cotton seed oil (:labeling name (INCI: Gossypium Herbaceum (Cotton) Seed Oil)), coconut oil (:labeling name (INCI: Cocos Nucifera (Coconut) Oil)), hydrogenated coconut oil (:labeling name (INCI: Hydrogenated Coconut Oil)), egg yolk oil (:labeling name (INCI: Egg Oil)), and the like.

### - Ester

Esters include liquid oils obtained by condensation between fatty acid having 1 to 20 carbon atoms and alcohol having 1 to 20 carbon atoms, such as polyesters including mono-, di-, and tri-esters. Specific examples thereof include: diisobutyl adipate (:labeling name (INCI: Diisobutyl Adipate)), dihexyldecyl adipate (:labeling name), diheptylundecyl adipate (:labeling name (INCI: Diheptylundecyl Adipate)), n-alkyl glycol monoisostearate such as isostearyl isostearate (:labeling name (INCI: Isostearyl Isostearate)), isocetyl isostearate (:labeling name (INCI: Isocetyl Isostearate)), trimethylolpropane triisostearate (:labeling name (INCI: Trimethylolpropane Triisostearate)), glycol diethylhexanoate (:labeling name (INCI: Glycol Diethylhexanoate)), cetyl ethylhexanoate (:labeling name (INCI: Cetyl Ethylhexanoate)), triethylhexanoin (:labeling name (INCI: Triethylhexanoin)), trimethylolpropane triethylhexanoate (:labeling name (INCI: Trimethylolpropane Triethylhexanoate)), pentaerythrityl tetraethylhexanoate (:labeling name (INCI: Pentaerythrityl Tetraethylhexanoate)), cetyl octanoate (:labeling name (INCI: Cetyl Ethylhexanoate)), octyldodecyl esters such as octyldodecyl stearoyl stearate (:labeling name (INCI: Octyldodecyl Stearoyl Stearate)), oleyl oleate (:labeling name (INCI: Oleyl Oleate)), octyldodecyl oleate (:labeling name (INCI: Octyldodecyl Oleate)), decyl oleate (:labeling name (INCI: Decyl Oleate)), neopentyl glycol dioctanoate (:labeling name (INCI: Neopentyl Glycol Diethylhexanoate)), neopentyl glycol dicaprate (:labeling name (INCI: Neopentyl Glycol Dicaprate)), triethyl citrate (:labeling name (INCI: Triethyl Citrate)), diethylhexyl succinate (:labeling name (INCI: Diethylhexyl Succinate)), amyl acetate (:labeling name (INCI: Amyl Acetate)), ethyl acetate (:labeling name (INCI: Etyl Acetate)), butyl acetate (:labeling name (INCI: Butyl Aceetate)), isocetyl stearate (:labeling name (INCI: Isocetyl Stearate)), butyl stearate (:labeling name (INCI: Butyl Stearate)), diisopropyl sebacate (:labeling name (INCI: Diisopropyl Sebacate)), diethylhexyl sebacate (:labeling name (INCI: Diethylhexyl Sebacate)), cetyl lactate (:labeling name (INCI: Cetyl Lactate)), myristyl lactate (:labeling name (INCI: Myristyl Lactate)), isononyl isononanoate (:labeling name (INCI: Isononyl Isononanoate)), isotridecyl isononanoate (:labeling name (INCI: Isotridecyl Isononanoate)); palmitates such as isopropyl palmitate (:labeling name (INCI: Isopropyl Palmitate)), ethylhexyl palmitate (:labeling name (INCI: Ethylhexyl Isopalmitate)), and hexyldecyl palmitate (:labeling name (INCI: Isocetyl Palmitate, Hexyldecyl Palmitate)); cholesteryl hydroxystearate (:labeling name (INCI: Cholesteryl Hydroxystearate)); myristates such as isopropyl myristate (:labeling name (INCI: Isopropyl Myristate)), octyldodecyl myristate (:labeling name (INCI: Octyldodecyl Myristate)), and myristyl myristate (:labeling name (INCI: Myristyl Myristate)); ethylhexyl laurate (:labeling name (INCI: Ethylhexyl Laurate)), hexyl laurate (:labeling name (INCI: Hexyl Laurate)), dioctyldodecyl lauroyl glutamate (:labeling name (INCI: Dioctyldodecyl Lauroyl Glutamate)), isopropyl lauroyl sarcosinate (:labeling name (INCI: Isopropyl Lauroyl Sarcosinate)), diisostearyl malate (:labeling name (INCI: Diisostearyl Malate)); and glyceride oils such as glyceryl acetate (:labeling name (INCI: Glyceryl Acetate)) and glyceryl stearate (:labeling name (INCI: Glyceryl Stearate)).

### - Silicone Oil

Examples of silicone oils include: trisiloxane (:labeling name (INCI: Trisiloxane)), volatile dimethicone (:labeling name (INCI: Dimethicone)), low viscosity dimethicone (:labeling name (INCI: Dimethicone)), cyclotetrasiloxane (:labeling name (INCI: Cyclotetrasiloxane)), cyclopentasiloxane (:labeling name (INCI: Cyclopentasiloxane)), cyclohexasiloxane (:labeling name (INCI: Cyclohexasiloxane)), methyl trimethicone (:labeling name (INCI: Methyl Trimethicone)), caprylyl methicone (:labeling name (INCI: Caprylyl Methicone)), phenyl trimethicone (:labeling name (INCI: Phenyl Trimethicone)), methylphenyl polysiloxane (:labeling name (INCI: Diphenyl Dimethicone)), diphenylsiloxy phenyl trimethicone (:labeling name (INCI: Diphenylsiloxy Phenyl Trimethicone)), ethyl methicone (:labeling name (INCI: Ethyl Methicone)), ethyl trisiloxane (:labeling name (INCI: Ethyl Trisiloxane)), linear or branched dimethicone having a low or high viscosity such as hydrogen dimethicone (:labeling name (INCI: Hydrogen Dimethicone)), amodimethicone (:labeling name (INCI: Amodimethicone)), aminopropyl dimethicone (:labeling name (INCI: Aminopropyl Dimethicone)); silicone gums such as dimethicone (:labeling name (INCI: Dimethicone)) gum with a high polymerization degree, amodimethicone (:labeling name (INCI: Amodimethicone)) gum, and dimethylsiloxane-methylphenylsiloxane copolymer gum; silicone gum or rubber in a cyclic organopolysiloxane solution, amino acid-modified silicone, fluorine-modified silicone, silicone resin and dissolved silicone resin, and the like.

### - Fluorine Oil

Fluorine oils include: perfluoropolyethers such as polyperfluoromethylisopropyl ether (:labeling name (INCI: Polyperfluoromethylisopropyl Ether)); and perfluorocarbons such as perfluorodecalin (:labeling name (INCI: Perfluorodecalin)) and perfluorohexane (:labeling name (INCI: Perfluorohexane)).

As (B) the oil in a liquid state at 25°C, silicone oils are preferred, among which methyl trimethicone (:labeling name (INCI: Methyl Trimethicone)), trisiloxane (:labeling name (INCI: Trisiloxane)), volatile dimethicone (:labeling name (INCI: Dimethicone)), dimethicone having kinematic viscosity of 0.5 to 20 mm²/s at 25°C (:labeling name (INCI: Dimethicone)), or diphenylsiloxy phenyl trimethicone (:labeling name (INCI: Diphenylsiloxy Phenyl Trimethicone)) is more preferred.

### Other Components

Components other than the above components can optionally be added to the cosmetic of the present invention in an appropriate amount. Examples of these optional components include (1) an oily component in a solid or semi-solid state at 25°C, (2) a compound having an alcoholic hydroxyl group, (3) a surfactant, (4) a composition including crosslinked organopolysiloxane and oil in a liquid state at 25°C, (5) a film-forming agent, (6) an antiperspirant, (7) an antibacterial agent, and (8) other additives. These can be used alone or in combination of two or more.

### (1) Oily Component in Solid or Semi-Solid State at 25°C

The oily component in a solid or semi-solid state at 25°C may be either in a solid or semi-solid state. For example, higher fatty acids, higher alcohols, esters, silicone oils, fluorine oils, and the like can be used.

### - Solid Oily Component

In the present invention, when it is desired to solidify the cosmetic, it is preferable to add waxes, hydrocarbons, esters, higher alcohols, or higher fatty acids in a solid state at 25°C. The oily component in a solid state at 25°C includes waxes, hydrocarbons, esters, higher alcohols, and higher fatty acids each having a melting point of preferably equal to or higher than 40°C, more preferably 60 to 110°C, although not particularly limited as long as it is an ingredient that can generally be added to a cosmetic. Specific examples thereof include: vegetable waxes such as carnauba wax, candelilla wax, rice bran wax, Japan wax, and shea butter; animal waxes such as beeswax and spermaceti; hydrocarbon waxes such as solid paraffin, polyethylene, ceresin, ozokerite, and microcrystalline wax; higher alcohols such as stearyl alcohol, behenyl alcohol, and cetanol; fatty acids such as stearic acid and behenic acid; and silicone waxes such as acrylic silicone resin in a form of an acrylic-silicone graft or block copolymer (acrylic-silicone graft copolymer: KP-561P, 562P, etc. produced by Shin-Etsu Chemical Co., Ltd.), or derivatives thereof.

### - Semi-Solid Oily Component

In the present invention, when it is desired to provide the cosmetic with a coating property and thickness, it is preferable to add hydrocarbons or esters in a semi-solid (paste) state at 25°C without particular limitation as long as they are ingredients that can generally be added to a cosmetic. Specific examples thereof include lanolin, petroleum jelly, dipentaerythritol fatty acid ester, hydrogenated oil, and the like.

### (2) Compound Having Alcoholic Hydroxyl Group

The compound having an alcoholic hydroxyl group includes: lower alcohols preferably having 2 to 5 carbon atoms, such as ethanol and isopropanol; sugar alcohols such as sorbitol and maltose; sterols such as cholesterol, sitosterol, phytosterol, and lanosterol; and the like. Note that the higher alcohols illustrated with regard to the component (1) are not included in this compound having an alcoholic hydroxyl group.

### (3) Surfactant

The surfactant includes, but is not particularly limited to, nonionic, anionic, cationic, and amphoteric surfactants. Any surfactants used for a common cosmetic can be used. Among these surfactants, partially crosslinked polyether-modified silicone, partially crosslinked polyglycerin-modified silicone, linear or branched polyoxyethylene-modified organopolysiloxane, linear or branched polyoxyethylene polyoxypropylene-modified organopolysiloxane, linear or branched polyoxyethylene/alkyl co-modified organopolysiloxane, linear or branched polyoxyethylene polyoxypropylene/alkyl co-modified organopolysiloxane, linear or branched polyglycerin-modified organopolysiloxane, linear or branched polyglycerin/alkyl co-modified organopolysiloxane, and pyrrolidone-modified organopolysiloxane are preferred. In these surfactants, an amount of a hydrophilic polyoxyethylene group, a polyoxyethylene polyoxypropylene group, or a polyglycerin residue is preferably 10 to 70% by mass of the molecule. Furthermore, when partially crosslinked polyether-modified silicone or partially crosslinked polyglycerin-modified silicone is used, it is preferred that in the composition including crosslinked organopolysiloxane and oil in a liquid state at 25°C, the crosslinked organopolysiloxane swells in the liquid oil by containing an amount of the own weight or more of the liquid oil. Liquid silicones, hydrocarbon oils, ester oils, natural animal or vegetable oils, semi-synthetic oils, or fluorine oils among the oils as the optional components can be used as the liquid oil. Examples thereof include: low viscosity silicone having kinematic viscosity of 0.65 to 100 mm²/s at 25°C; hydrocarbon oils such as liquid paraffin, squalane, isododecane, and isohexadecane; glyceride oils such as trioctanoin; ester oils such as isotridecyl isononanoate, N-acylglutamate, and lauroyl sarcosinate; and natural animal or vegetable oils such as macadamia nut oil. Specific examples thereof include KSG-210, KSG-240, KSG-310, KSG-320, KSG-330, KSG-340, KSG-320Z, KSG-350Z, KSG-710, KSG-810, KSG-820, KSG-830, KSG-840, KSG-820Z, KSG-850Z, etc. produced by Shin-Etsu Chemical Co., Ltd. Specific examples of the surfactant other than crosslinked organopolysiloxane include KF-6011, KF-6013, KF-6043, KF-6017, KF-6028, KF-6038, KF-6048, KF-6100, KF-6104, KF-6105, KF-6106, etc. produced by Shin-Etsu Chemical Co., Ltd. In any case, the amount of the surfactant contained in the cosmetic is preferably 0.1 to 20% by mass. When the amount is equal to or more than 0.1% by mass, functions of dispersion and emulsification can sufficiently be exerted. The amount is preferably equal to or less than 20% by mass because there is no risk that the cosmetic has a sticky use feeling. Although HLB of the surfactant is not limited, it is preferably 2 to 14.5 in order to maintain water resistance of the cosmetic.

### (4) Composition Including Crosslinked Organopolysiloxane and Oil in Liquid State at 25°C

In the composition including crosslinked organopolysiloxane and oil in a liquid state at 25°C, it is preferred that the crosslinked organopolysiloxane swells in the liquid oil by containing an amount of the own weight or more of the liquid oil. Liquid silicones, hydrocarbon oils, ester oils, natural animal or vegetable oils, semi-synthetic oils, or fluorine oils among the oils as the optional components can be used. Examples thereof include: low viscosity silicone having kinematic viscosity of 0.65 to 100 mm²/s at 25°C; hydrocarbon oils such as liquid paraffin, squalane, isododecane, and isohexadecane; glyceride oils such as trioctanoin; ester oils such as isotridecyl isononanoate, N-acylglutamate, and lauroyl sarcosinate; and natural animal or vegetable oils such as macadamia nut oil. The component (4) is different from the component (3) according to the present invention, and is a compound having no polyether or polyglycerol structure in the molecular structure. Specific examples thereof include KSG series (product name) produced by Shin-Etsu Chemical Co., Ltd., particularly KSG-15, KSG-16, KSG-016F, KSG-19, KSG-41, KSG-42, KSG-43, KSG-44, KSG-042Z, KSG-045Z, etc.

### (5) Film-Forming Agent

The film-forming agent is added mainly for the purpose of further prolonging the sustained effect of the cosmetic. Although not particularly limited, it is preferably a silicone composition from the viewpoint of imparting water repellency. Specifically, trimethylsiloxysilicate, acrylic-silicone film former, silicone-modified norbornene, silicone-modified pullulan, and the like can be used. The film-forming agent may be dissolved in oil in a liquid state at room temperature in advance, and then added to the cosmetic. Liquid silicones, hydrocarbon oils, ester oils, natural animal or vegetable oils, semi-synthetic oils, or fluorine oils among the oils as the optional components can be used as the oil in a liquid state. Examples thereof include: low viscosity silicone having kinematic viscosity of 0.65 to 100 mm²/s at 25°C; hydrocarbon oils such as liquid paraffin, squalane, isododecane, and isohexadecane; glyceride oils such as trioctanoin; ester oils such as isotridecyl isononanoate, N-acylglutamate, and lauroyl sarcosinate; and natural animal or vegetable oils such as macadamia nut oil. Additionally, specific examples thereof include: KF-7312J, which is trimethylsiloxysilicate dissolved in silicone; KP-545 and KP-549, each of which is an acrylic-silicone film former dissolved in silicone; NBN-30-ID, which is silicone-modified norbornene dissolved in isododecane; TSPL-30-ID, which is silicone-modified pullulan dissolved in isododecane; TSPL-30-D5, which is dissolved in silicone; etc., each produced by Shin-Etsu Chemical Co., Ltd.

### (6) Antiperspirant

When the cosmetic according to the present invention is a deodorant, an antiperspirant can optionally be added. The antiperspirant is not particularly limited as long as it is a component that tightens skin to suppress sweat, and general-purpose components can widely be used. Examples thereof include aluminum chlorohydrate, aluminum chloride, aluminum chlorohydroxy allantoinate, aluminum salt of allantoin, tannic acid, aluminum potassium sulfate, zinc oxide, zinc para-phenolsulfonate, burnt alum, tetrachloro (Al/zirconium) hydride, trichlorohydrex glycine (Al/zirconium), and the like. Particularly, an antiperspirant active ingredient selected from a group consisting of aluminum halides, aluminum hydroxyhalides, and complexes or mixtures thereof with zirconyl oxyhalides and zirconyl hydroxyhalides is preferred as a component exerting a high effect. These antiperspirants for use can be added after dissolved in water, or can be added in a powder form directly to a preparation. Commercial products can also be used as the antiperspirant. The commercial product to be used may be in a form of an ingredient mixed with other components. An amount of the antiperspirant is not particularly limited, and can appropriately be changed according to the amounts of other components. To obtain a deodorant having an excellent antiperspirant effect and to obtain a deodorant with reduced skin irritation, the amount thereof contained in the cosmetic is preferably 0.001 to 30% by mass, more preferably 0.01 to 20% by mass.

### (7) Antibacterial Agent

The antibacterial agent is not particularly limited as long as it is a component capable of providing a deodorizing effect by suppressing growth of normal flora on the skin, which produces substances causing a body odor. For example, commonly used antibacterial agents include triclosan, benzalkonium chloride, benzethonium chloride, chlorhexidine hydrochloride, chlorhexidine gluconate, halocarban, isomethylphenol, and the like. Furthermore, essential oils or extracts from crude drugs having antibacterial effects, such as camellia sinensis leaf extract, may also be added. Usable as the antibacterial agent such as essential oils or extracts from crude drugs having a deodorizing effect is, for example, green tea extract, lavandula angustifolia extract, scutellaria baicalensis root extract, coptis japonica extract, plantago asiatica extract, artemisia capillaris extract, aloe arborescens extract, sophora angustifolia root extract, sasa veitchii leaf extract, allium sativum extract, hamamelis virginiana extract, black tea extract, salvia officinalis leaf extract, zanthoxylum piperitum extract, zingiber officinale root extract, acorus calamus root extract, hedera helix extract, houttuynia cordata extract, prunus persica fruit extract, peach leaf extract, mentha piperita leaf extract, cnidium officinale root extract, eucalyptus globulus leaf extract, arachis hypogaea seedcoat extract, ganoderma lucidum extract, sanguisorba officinalis root extract, and the like.

### (8) Other Additives

Other additives include oil-soluble gelling agents, moisturizers, preservatives, fragrances, salts, antioxidants, pH adjusters, chelating agents, refreshing agents, antiinflammatory agents, skin-beautifying ingredients (whitening agents, cell activators, skin roughness improvement agents, blood circulation promoters, skin astringents, antiseborrheic agents, etc.), vitamins, amino acids, nucleic acids, hormones, inclusion compounds, and the like.

### - Oil-Soluble Gelling Agent

Oil-soluble gelling agents include: metal soaps such as organo-modified bentonite, aluminum stearate, magnesium stearate, and zinc myristate; amino acid derivatives such as N-lauroyl-L-glutamic acid and α,γ-di-n-butylamine; dextrin fatty acid esters such as dextrin palmitate, dextrin stearate, and dextrin 2-ethylhexanoate palmitate; sucrose fatty acid esters such as sucrose palmitate and sucrose stearate; fructooligosaccharide fatty acid esters such as fructooligosaccharide stearate and fructooligosaccharide 2-ethylhexanoate; benzylidene derivatives of sorbitol such as monobenzylidene sorbitol and dibenzylidene sorbitol; and the like.

### - Moisturizer

Moisturizers include glycerin, sorbitol, propylene glycol, dipropylene glycol, 1,3-butylene glycol, pentylene glycol, glucose, xylitol, maltitol, polyethylene glycol, hyaluronic acid, chondroitin sulfate, pyrrolidone carboxylate, polyoxyethylene methyl glucoside, polyoxypropylene methyl glucoside, yolk lecithin, soybean lecithin, phosphatidylcholine, phosphatidylethanolamine, phosphatidylserine, phosphatidylglycerol, phosphatidylinositol, sphingolipid, and the like.

### - Preservative

Preservatives include alkyl esters of parahydroxybenzoic acid, benzoic acid, sodium benzoate, sorbic acid, potassium sorbate, phenoxyethanol, and the like. Antibacterial agents include benzoic acid, salicylic acid, carbolic acid, sorbic acid, alkyl esters of parahydroxybenzoic acid, parachlorometacresol, hexachlorophene, trichlorocarbanilide, photosensitizers, and the like.

### - Fragrance

Fragrances include natural fragrances and synthetic fragrances. The natural fragrances include: plant fragrances isolated from flowers, leaves, wood, and pericarps; and animal fragrances such as musk and civet. The synthetic fragrances include: hydrocarbons such as monoterpene; alcohols such as aliphatic alcohols and aromatic alcohols; aldehydes such as terpene aldehydes and aromatic aldehydes; ketones such as alicyclic ketones; esters such as terpene esters; lactones; phenols; oxides; nitrogen-containing compounds; acetals; and the like.

### - Salts

Salts include inorganic salts, organic acid salts, amine salts, and amino acid salts. Examples of the inorganic salts include sodium salt, potassium salt, magnesium salt, calcium salt, aluminum salt, zirconium salt, zinc salt, and the like of inorganic acids such as hydrochloric acid, sulfuric acid, carbonic acid, and nitric acid. Examples of the organic acid salts include salts of organic acids such as acetic acid, dehydroacetic acid, citric acid, malic acid, succinic acid, ascorbic acid, and stearic acid. Examples of the amine salts and amino acid salts include salts of amines such as triethanolamine and salts of amino acids such as glutamic acid. Furthermore, other salts such as salts of hyaluronic acid, chondroitin sulfuric acid, and the like, aluminum zirconium glycine complexes, and further acid-alkali neutralizing salts used in prescription of a cosmetic can also be used.

### - Antioxidant

Examples of antioxidants include, but are not particularly limited to, carotenoid, ascorbic acid and a salt thereof, ascorbyl stearate, tocopherol acetate, tocopherol, p-t-butylphenol, butylhydroxyanisol, dibutylhydroxytoluene, phytic acid, ferulic acid, thiotaurine, hypotaurine, sulfite, erythorbic acid and a salt thereof, chlorogenic acid, epicatechin, epigallocatechin, epigallocatechin gallate, apigenin, kaempferol, myricetin, quercetin, and the like.

### - pH Adjuster

pH adjusters include lactic acid, citric acid, glycolic acid, succinic acid, tartaric acid, dl-malic acid, potassium carbonate, sodium bicarbonate, ammonium bicarbonate, and the like.

### - Chelating Agent

Chelating agents include alanine, sodium edetate, sodium polyphosphate, sodium metaphosphate, phosphoric acid, aspartic diacetic acid, ethylenediamine disuccinic acid, and the like.

### - Refreshing Agent

Refreshing agents include L-menthol, camphor, and the like.

### - Antiinflammatory Agent

Antiinflammatory agents include allantoin, glycyrrhizic acid and a salt thereof, glycyrrhetinic acid and stearyl glycyrrhetinate, tranexamic acid, azulene, and the like.

### - Skin-Beautifying Ingredient

Skin-beautifying ingredients include: whitening agents such as vitamin C derivatives, hydroquinone, tranexamic acid, arbutin, phenylethyl resorcinol, kojic acid, and plant extracts; cell activators such as royal jelly, photosensitizers, cholesterol derivatives, and deproteinized calf blood extract; skin roughness improvement agents; blood circulation promoters such as nonanoic acid vanillylamide, benzyl nicotinate, β-butoxyethyl nicotinate, capsaicin, zingerone, cantharides tincture, ichthammol, caffeine, tannic acid, α-borneol, tocopherol nicotinate, inositol hexanicotinate, cyclandelate, cinnarizine, tolazoline, acetylcholine, verapamil, cepharanthine, and γ-oryzanol; skin astringents such as zinc oxide and tannic acid; antiseborrheic agents such as sulfur and thianthrol; and the like.

### - Vitamins

Vitamins include: vitamin A family such as vitamin A oil, retinol, retinol acetate, and retinol palmitate; vitamin B family including vitamin B2 family such as riboflavin, riboflavin butyrate, and flavin adenine nucleotide, vitamin B6 family such as pyridoxine hydrochloride, pyridoxine dioctanoate, and pyridoxine tripalmitate, vitamin B12 and derivatives thereof, and vitamin B15 and derivatives thereof; vitamin C family such as L-ascorbic acid, L-ascorbic acid dipalmitate, sodium L-ascorbic acid-2-sulfate, and dipotassium L-ascorbic acid diphosphate; vitamin D family such as ergocalciferol and cholecalciferol; vitamin E family such as α-tocopherol, β-tocopherol, γ-tocopherol, dl-α-tocopherol acetate, dl-α-tocopherol nicotinate, and dl-α-tocopherol succinate; nicotinic acids such as nicotinic acid, benzyl nicotinate, and amide nicotinate; vitamin H; vitamin P; pantothenic acids such as calcium pantothenate, D-pantothenyl alcohol, pantothenyl ethyl ether, and acetyl pantothenyl ethyl ether; biotin; and the like.

### - Amino Acids

Amino acids include glycine, valine, leucine, isoleucine, serine, threonine, phenylalanine, arginine, lysine, aspartic acid, glutamic acid, cystine, cysteine, methionine, tryptophan, and the like.

### - Nucleic Acid

Nucleic acids include deoxyribonucleic acid and the like.

### - Hormone

Hormones include estradiol, ethenyl estradiol, and the like.

### - Inclusion Compound

Inclusion compounds include cyclodextrin and the like.

### EXAMPLE

Hereinafter, the present invention will be specifically described with reference to Examples and Comparative Examples. However, the present invention is not limited thereto.

Incidentally, unless otherwise especially noted, "%" in the composition means "mass%" in the below examples. Viscosity means a measured value of viscosity at 25°C determined by a rotational viscometer described in JIS K 7117-1:1999.

### Preparation Examples 1 to 4, Silicone-Modified Clay Minerals 1 to 2

### Preparation Example 1

2 g of refined bentonite (product name: Kunipia-F, produced by KUNIMINE), which is a water-swellable clay mineral, was dispersed in 200 g of aqueous solution, and the solution was heated and stirred at 60°C while 2 g of ammonium group-modified organopolysiloxane represented by the following formula (A-1) dissolved in 200 g of isopropyl alcohol was added dropwise. After stirring for two hours, the solution was removed by filtration and dried under reduced pressure, thereby obtaining a clay mineral treated with the ammonium group-modified organopolysiloxane.

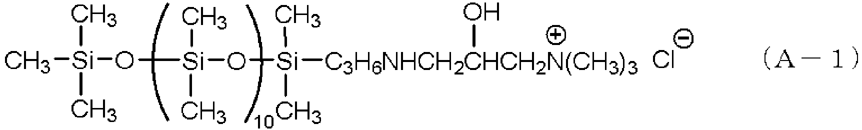

### Preparation Example 2

A clay mineral treated with ammonium group-modified organopolysiloxane was obtained in the same manner as in Preparation Example 1 except that ammonium group-modified organopolysiloxane represented by the following formula (A-2) was used instead of the ammonium group-modified organopolysiloxane (A-1) used for Preparation Example 1.

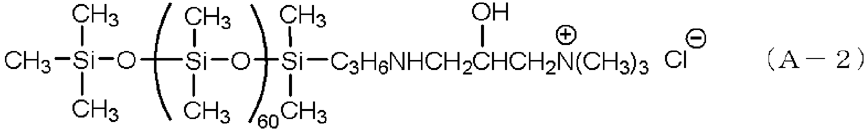

### Preparation Example 3

A clay mineral treated with ammonium group-modified organopolysiloxane was obtained in the same manner as in Preparation Example 1 except that ammonium group-modified organopolysiloxane represented by the following formula (A-3) was used instead of the ammonium group-modified organopolysiloxane (A-1) used for Preparation Example 1.

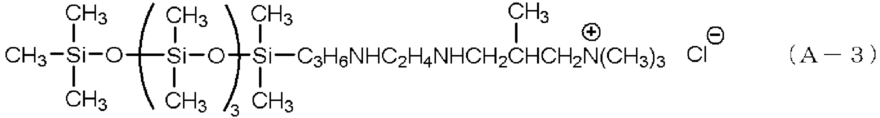

### Preparation Example 4

A clay mineral treated with ammonium group-modified organopolysiloxane was obtained in the same manner as in Preparation Example 1 except that ammonium group-modified organopolysiloxane represented by the following formula (A-4) was used instead of the ammonium group-modified organopolysiloxane (A-1) used for Preparation Example 1.

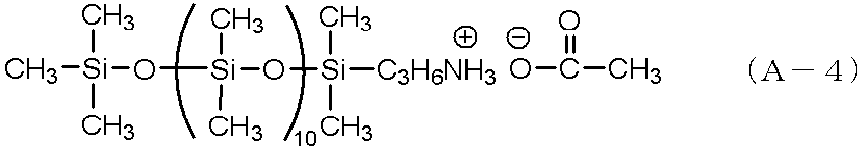

### Silicone-Modified Clay Mineral 1

A silicone-modified clay mineral 1 was obtained in the same manner as in Preparation Example 1 except that ammonium group-modified organopolysiloxane represented by the following formula (A-1)' was used instead of the ammonium group-modified organopolysiloxane (A-1) used for Preparation Example 1.

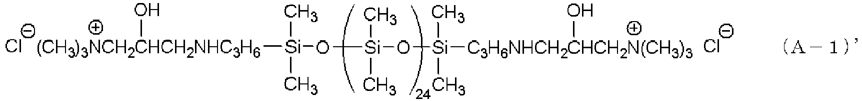

### Silicone-Modified Clay Mineral 2

A silicone-modified clay mineral 2 was obtained in the same manner as in Preparation Example 1 except that ammonium group-modified organopolysiloxane represented by the following formula (A-2)' was used instead of the ammonium group-modified organopolysiloxane (A-1) used for Preparation Example 1.

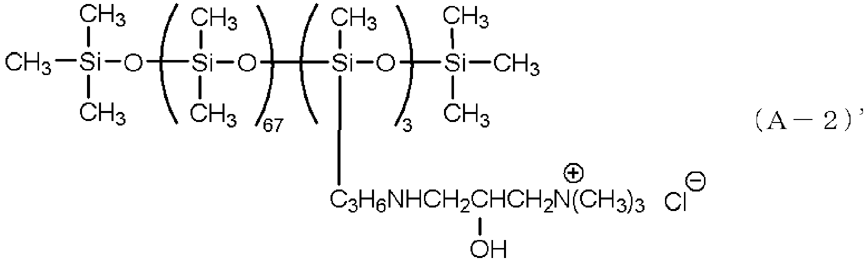

### Examples 1 to 4, Comparative Examples 1 to 5

Each component in an amount shown in Table 1 was mixed according to the following production method to obtain a gelled composition.

**Table 1**

| Composition | | Ex. 1 | Ex. 2 | Ex. 3 | Ex. 4 | Comp. Ex.1 | Comp. Ex.2 | Comp. EX.3 | Comp. Ex.4 | Comp. Ex.5 |
|---|---|---|---|---|---|---|---|---|---|---|
| A | Preparation Example.1 | 2 | | | | | | | | |
| | Preparation Example.2 | | 2 | | | | | | | |
| | Preparation Example.3 | | | 2 | | | | | | |
| | Preparation Example.4 | | | | 2 | | | | | |
| | Silicone-modified clay mineral 1 | | | | | | 2 | | | |
| | Silicone-modified clay mineral 2 | | | | | | | 2 | | |
| | Bentonite (*) | | | | | | | | 2 | |
| | Quaternium-18 Bentonite (*) | | | | | | | | | 2 |
| B | Cyclopenta Siloxane (*) | 8 | 8 | 8 | | 10 | 8 | 8 | 8 | 8 |
| | Dimethylpoly Siloxane (*) | | | | 8 | | | | | |
| PEG-10 Dimethicone (*) | | 1 | 1 | 1 | | 1 | 1 | 1 | 1 | 1 |
| Propyl carbonate | | | | | 1 | | | | | |
| Appearance (uniformity) | | Good | Good | Good | Good | Poor | Fair | Good | Poor | Poor |
| Stability over time at 50°C | | Very Good | Good | Very Good | Good | - | - | Poor | - | - |
| Viscosity [mPa's] | | Very Good | Very Good | Very Good | 4100 | - | - | 2900 | - | - |

| | | | | | | | | | | |
|---|---|---|---|---|---|---|---|---|---|---|
| Components (*) Bentonite: Kunipia-F (produced by KUNIMINE INDUSTRIES CO., LTD.) (*) Quaternium-18 Bentonite: Moistnite-WO (produced by KUNIMINE INDUSTRIES CO., LTD.) (*) Cyclopentasiloxane: KF-995 (kinematic viscosity: 4 mm²/s, produced by Shin-Etsu Chemical Co., Ltd.) (*) Dimethylpolysiloxane: KF-96A-6cs (kinematic viscosity: 6 mm²/s, produced by Shin-Etsu Chemical Co., Ltd.) (*) PEG-10 Dimethicone: KF-6017 (produce by Shin-Etsu Chemical Co., Ltd.) | | | | | | | | | | |

### (Production Method)

The component (B) in Table 1 was stirred by a disper while being heated at 60°C, and the component (A) was added portionwise thereto. Next, PEG-10 Dimethicone or propyl carbonate was added as necessary and stirred for 10 minutes, thereby obtaining a gelled composition.

The gelled compositions of Examples 1 to 4 and Comparative Examples 1 to 5 were evaluated according to the following evaluation method for appearance (uniformity), appearance (stability over time at 50°C), and viscosity of the composition. The results are shown in Table 1.

### Appearance (Uniformity)

25 g of the gelled composition was placed in a glass bottle, visually observed whether liquid phase separation and agglomeration or precipitation of the clay mineral occurred, and evaluated based on the below criteria.

### (Evaluation Criteria)

Good: no agglomeration of powder nor oil float
Fair: some agglomeration of powder or oil float observed
Poor: powder and oil were completely separated from each other

### Stability over Time at 50°C

50 g of the gelled composition was placed in a glass bottle and stored at 50°C for 1 week. Thereafter, it was visually observed whether liquid phase separation and agglomeration of the gelled composition occurred, and evaluated based on the below criteria.

### (Evaluation Criteria)

Very Good: no change from immediately after the preparation of the composition
Good: some oil float occurred
Poor: powder and oil were completely separated

### Viscosity

50 g of the gelled composition was placed in a glass bottle and viscosity at 25°C thereof was measured by a method using a rotational viscometer described in JIS K 7117-1:1999. Note that the result is shown as "Very good" for the viscosity that could not be measured because the sample had lost fluidity and solidified.

As can clearly be seen from the above results, it was found that Examples 1 to 4 all yielded a gelled composition of silicone oil having excellent uniform appearance and stability over time.

On the other hand, gelation was not confirmed in Comparative Example 1 containing no clay minerals.

It was found that Comparative Examples 2 and 3 respectively containing a clay mineral treated with dual-end type ammonium group-modified organopolysiloxane and a clay mineral treated with side-chain type ammonium group-modified organopolysiloxane resulted in low viscosity of the composition, as well as decreased uniformity of appearance and stability over time.

Furthermore, it was found that Comparative Examples 4 and 5 respectively using an untreated clay mineral and an organo-modified clay mineral resulted in extreme agglomeration of the clay mineral in the composition and decreased uniformity of appearance.

Accordingly, it was found that the organopolysiloxane of the present invention can favorably gelate silicone oil and provide a gelled composition having excellent uniformity and stability over time.

### Examples 5 to 7

### Example 5: W/O Cream

| (Components) | (%) |
|---|---|
| 1. Dimethylpolysiloxane (*) | 10.0 |
| 2. Cyclopentasiloxane | 7.0 |
| 3. Glyceryl trioctanoate | 4.5 |
| 4. Polyether-modified branched si licone (*) | 2.0 |
| 5. Preparation Example 1 | 1.5 |
| 6. Dipropylene glycol | 7.0 |
| 7. Preservative | Moderate amount |
| 8. Fragrance | Moderate amount |
| 9. Purified water | Rest |
| Total | 100.0 |

| | |
|---|---|
| (*) Dimethylpolysiloxane: KF-96A-6cs (produced by Shin-Etsu Chemical Co., Ltd.) (*) Polyether-modified branched silicone: KF-6028 (produced by Shin-Etsu Chemical Co., Ltd.) A) Components 1 to 5 were mixed. B) Components 6 to 9 were mixed and added to the above A), and stirred and emulsified. | |

The W/O cream thus obtained was not greasy or sticky, lightly and freshly spread and expanded, had excellent adhesion, and also provided a natural finish. Furthermore, excellent stability of the W/O cream was also confirmed.

### Example 6: W/O Cream

| (Components) | (%) |
|---|---|
| 1. Preparation Example 2 | 2.0 |
| 2. Dimethylpolysiloxane (*) | 10.0 |
| 3. Crosslinked polyether-modified silicone (*) | 5.0 |
| 4. Dipropylene glycol | 10.0 |
| 5. Sodium citrate | 0.2 |
| 6. Ethanol | 5.0 |
| 7. Preservative | Moderate amount |
| 8. Fragrance | Moderate amount |
| 9. Purified water | Rest |
| Total | 100.0 |

| | |
|---|---|
| (*) Dimethylpolysiloxane: KF-96A-6cs (produced by Shin-Etsu Chemical Co., Ltd.) (*) Crosslinked polyether-modified silicone: KSG-21 (produced by Shin-Etsu Chemical Co., Ltd.) A) Components 1 to 3 were mixed. B) Components 4 to 9 were mixed and added to the above A), and stirred and emulsified. | |

The W/O cream thus obtained was not greasy or sticky, lightly and freshly spread and expanded, had excellent adhesion, and also provided a natural finish. Furthermore, excellent stability of the W/O cream was also confirmed.

### Example 7: Foundation

| (Components) | (%) |
|---|---|
| 1. Ceresin | |
| 2. Microcrystalline wax | 5.5 1.0 |
| 3. Liquid paraffin | 4.0 |
| 4. Propylene glycol dicaprylate | 3.0 |
| 5. Alkyl/polyether co-modified silicone (*) | 1.0 |
| 6. Alkyl-modified crosslinked polyether-modified silicone (*) | 7.0 |
| 7. Dimethylpolysiloxane (*) | 15.0 |
| 8. Preparation Example 1 | 1.5 |
| 9. Pigment | Moderate amount |
| 10. Oil-treated titanium oxide | 10.0 |
| 11. Lecithin | 0.3 |
| 12. Polyoxyethylene sorbitan monooleate | 0.5 |
| 13. 1,3-butylene glycol | 8.0 |
| 14. Preservative | Moderate amount |
| 15. Fragrance | Moderate amount |
| 16. Purified water | Rest |
| Total | 100.0 |

| | |
|---|---|
| (*) Alkyl/polyether co-modified silicone: KF-6026 (produced by Shin-Etsu Chemical Co., Ltd.) (*) Alkyl-modified crosslinked polyether-modified silicone: KSG-33 (produced by Shin-Etsu Chemical Co., Ltd.) (*) Dimethylpolysiloxane: KF-96-6cs (produced by Shin-Etsu Chemical Co., Ltd.) A) Components 1 to 8 were dissolved by heating. B) Components 9 to 12 and 14 were added to and homogeneously mixed with Component 13. C) Component 16 was added to the above B) and homogeneously dispersed. D) The above C) and Component 15 were added to the above A), stirred and emulsified, and filled to obtain a product. | |

It was confirmed that the foundation thus obtained did not produce oiliness or stickiness despite the presence of much oil, lightly spread and expanded, had excellent adhesion, provided a natural finish, and also achieved excellent long-lasting makeup.

According to the above results, it was demonstrated that the gelling agent of the present invention can yield a gelled composition having excellent uniform appearance and stability over time. Furthermore, it was demonstrated that a cosmetic including the gelling agent of the present invention is excellent in adhesion and provides a natural finish.

The present specification includes the following aspects.
[1]: A gelling agent comprising a water-swellable clay mineral in which ammonium group-modified organopolysiloxane represented by the following average composition formula (1) is incorporated in an interlayer space,

   (R¹₃SiO_{1/2})ₐ₁(R²ₙR¹₃₋ₙSiO_{1/2})ₐ₂(R¹₂SiO_{2/2})_{b}(R¹SiO_{3/2})_{c}(SiO_{4/2})_{d} (1)

   in the formula (1), R¹ independently represents a group selected from an alkyl group having 1 to 10 carbon atoms, an aryl group having 6 to 10 carbon atoms, and an arylene group having 7 to 10 carbon atoms,
   R² represents an ammonium group represented by the following formula (2),
   "a1" satisfies 0 < a1 ≤ 20, "a2" satisfies 0 < a2 ≤ 12, "b" satisfies 0 ≤ b ≤ 150, "c" satisfies 0 ≤ c ≤ 10, and "d" satisfies 0 ≤ d≤ 5, provided that a1 + a2 + b + c + d is 2 to 170; and "n" represents an integer of 1 to 3, 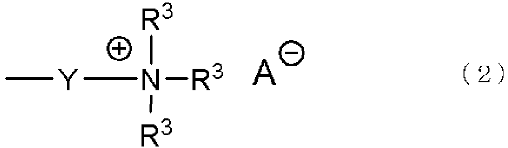
   in the formula (2), R³ independently represents a hydrogen atom or a group selected from an alkyl group having 1 to 20 carbon atoms, an aryl group having 6 to 10 carbon atoms, and an arylene group having 7 to 10 carbon atoms,
   Y represents a group represented by any of following formulae (3), and
   A⁻ represents an anion that is a counterion to an ammonium ion, 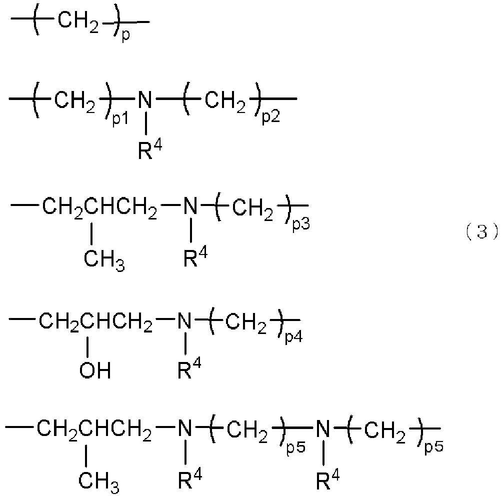
   wherein "p" represents an integer of 1 ≤ p ≤ 10; "p1" satisfies 1 ≤ p1 ≤ 10 and "p2" represents an integer of 1≤ p2 ≤10, provided 1≤ p1 + p2 ≤ 15; "p3" represents an integer of 1 ≤ p3 ≤ 10; "p4" represents an integer of 1 ≤ p4 ≤ 12; and "p5" represents an integer of 1 ≤ p5 ≤ 10, and
   R⁴ represents a hydrogen atom or a group selected from an alkyl group having 1 to 20 carbon atoms, an aryl group having 6 to 10 carbon atoms, and an arylene group having 7 to 10 carbon atoms.
[2]: The gelling agent according to [1], wherein the anion represented as A⁻ in the formula (2) is an anion selected from a hydroxide ion, a chloride ion, a bromide ion, a nitrate ion, a hydrogen sulfate ion, a hydrogen carbonate ion, and an acetate ion.
[3]: The gelling agent according to [1] or [2], wherein the ammonium group-modified organopolysiloxane is represented by the following formula (4), 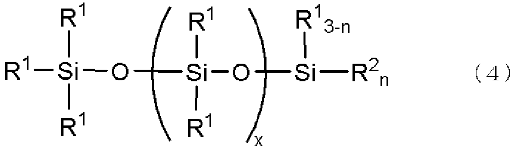 in the formula (4), R¹, R², and "n" are same as above; and "x" satisfies 0 ≤ x ≤ 100.
[4]: The gelling agent according to any one of [1] to [3], wherein the water-swellable clay mineral is one or more water-swellable clay minerals selected from montmorillonite, saponite, smectite, hectorite, sodium silicic mica, sodium tainiolite, or lithium tainiolite.
[5]: A cosmetic comprising the gelling agent according to any one of [1] to [4].
[6]: The cosmetic according to [5], further comprising oil in a liquid state at 25°C.
[7]: The cosmetic according to [6], wherein the oil comprises silicone oil.
[8]: A method for producing a gelling agent, comprising a step of:
   treating a water-swellable clay mineral with ammonium group-modified organopolysiloxane represented by the following average composition formula (1),

      (R¹₃SiO_{1/2})ₐ₁(R²ₙR¹₃₋ₙSiO_{1/2})ₐ₂(R¹SiO_{2/2})_{b}(R¹SiO_{3/2})_{c}(SiO_{4/2})_{d} (1)
   in the formula (1), R¹ independently represents a group selected from an alkyl group having 1 to 10 carbon atoms, an aryl group having 6 to 10 carbon atoms, and an arylene group having 7 to 10 carbon atoms,
   R² represents an ammonium group represented by the following formula (2),
   "a1" satisfies 0 < a1 ≤ 20, "a2" satisfies 0 < a2 ≤ 12, "b" satisfies 0 ≤ b ≤ 150, "c" satisfies 0 ≤ c ≤ 10, and "d" satisfies 0 ≤ d ≤ 5, provided that a1 + a2 + b + c + d is 2 to 170; and "n" represents an integer of 1 to 3, 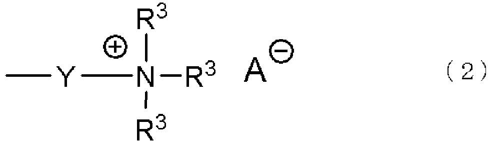
   in the formula (2), R³ independently represents a hydrogen atom or a group selected from an alkyl group having 1 to 20 carbon atoms, an aryl group having 6 to 10 carbon atoms, and an arylene group having 7 to 10 carbon atoms,
   Y represents a group represented by any of following formulae (3), and
   A⁻ represents an anion that is a counterion to an ammonium ion, 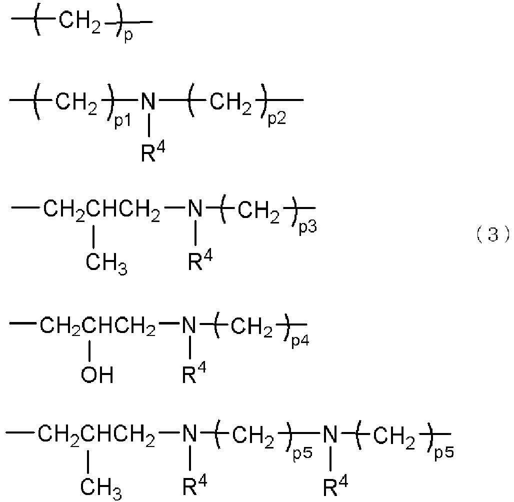
   wherein "p" represents an integer of 1≤p≤10; "p1" satisfies 1 ≤ p1 ≤ 10 and "p2" represents an integer of 1 ≤ p2 ≤ 10, provided 1 ≤ p1 + p2 ≤ 15; "p3" represents an integer of 1≤p3≤10; "p4" represents an integer of 1 ≤ p4 ≤ 12; and "p5" represents an integer of 1 ≤ p5 ≤ 10, and
   R⁴ represents a hydrogen atom or a group selected from an alkyl group having 1 to 20 carbon atoms, an aryl group having 6 to 10 carbon atoms, and an arylene group having 7 to 10 carbon atoms.
[9]: The method for producing a gelling agent according to [8], wherein the anion represented as A⁻ in the formula (2) is an anion selected from a hydroxide ion, a chloride ion, a bromide ion, a nitrate ion, a hydrogen sulfate ion, a hydrogen carbonate ion, and an acetate ion.
[10]: The method for producing a gelling agent according to [8] or [9], wherein a compound represented by the following formula (4) is used as the ammonium group-modified organopolysiloxane, 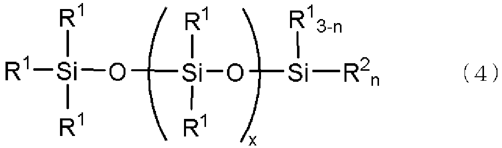 in the formula (4), R¹ ,R², and "n" are same as above; and "x" satisfies 0 ≤ x ≤ 100.
[11]: The method for producing a gelling agent according to any one of [8] to [10], wherein one or more water-swellable clay minerals selected from montmorillonite, saponite, smectite, hectorite, sodium silicic mica, sodium tainiolite, or lithium tainiolite are used as the water-swellable clay mineral.

It should be noted that the present invention is not limited to above-described embodiments. The embodiments are just examples, and any examples that substantially have the same feature and demonstrate the same functions and effects as those in the technical concept disclosed in claims of the present invention are included in the technical scope of the present invention.

## Claims

1. A gelling agent comprising a water-swellable clay mineral in which ammonium group-modified organopolysiloxane represented by the following average composition formula (1) is incorporated in an interlayer space,
(R¹₃SiO_{1/2})ₐ₁(R²ₙR¹₃₋ₙSiO_{1/2})ₐ₂(R¹₂SiO_{2/2})_{b}(R¹SiO_{3/2})_{c}(SiO_{4/2})_{d} (1)
in the formula (1), R¹ independently represents a group selected from an alkyl group having 1 to 10 carbon atoms, an aryl group having 6 to 10 carbon atoms, and an arylene group having 7 to 10 carbon atoms,
R² represents an ammonium group represented by the following formula (2),
"a1" satisfies 0 < a1 ≤ 20, "a2" satisfies 0 < a2 ≤ 12, "b" satisfies 0 ≤ b ≤ 150, "c" satisfies 0 ≤ c ≤ 10, and "d" satisfies 0 ≤ d≤ 5, provided that a1 + a2 + b + c + d is 2 to 170; and "n" represents an integer of 1 to 3, 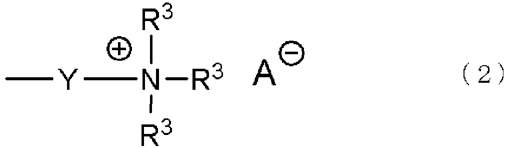
in the formula (2), R³ independently represents a hydrogen atom or a group selected from an alkyl group having 1 to 20 carbon atoms, an aryl group having 6 to 10 carbon atoms, and an arylene group having 7 to 10 carbon atoms,
Y represents a group represented by any of following formulae (3), and
A⁻ represents an anion that is a counterion to an ammonium ion, 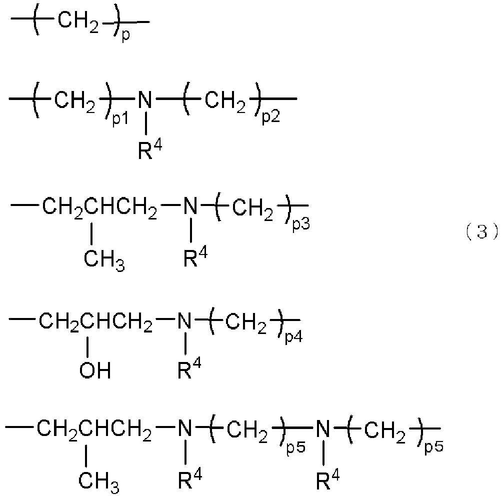
wherein "p" represents an integer of 1 ≤ p ≤ 10; "p1" satisfies 1 ≤ p1 ≤ 10 and "p2" represents an integer of 1≤ p2 ≤10, provided 1≤ p1 + p2 ≤ 15; "p3" represents an integer of 1 ≤ p3 ≤ 10; "p4" represents an integer of 1 ≤ p4 ≤ 12; and "p5" represents an integer of 1 ≤ p5 ≤ 10, and
R⁴ represents a hydrogen atom or a group selected from an alkyl group having 1 to 20 carbon atoms, an aryl group having 6 to 10 carbon atoms, and an arylene group having 7 to 10 carbon atoms.

2. The gelling agent according to claim 1, wherein the anion represented as A⁻ in the formula (2) is an anion selected from a hydroxide ion, a chloride ion, a bromide ion, a nitrate ion, a hydrogen sulfate ion, a hydrogen carbonate ion, and an acetate ion.

3. The gelling agent according to claim 1, wherein the ammonium group-modified organopolysiloxane is represented by the following formula (4), 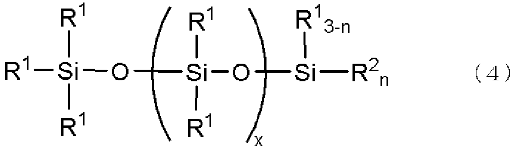 in the formula (4), R¹, R², and "n" are same as above; and "x" satisfies 0 ≤ x ≤ 100.

4. The gelling agent according to claim 1, wherein the water-swellable clay mineral is one or more water-swellable clay minerals selected from montmorillonite, saponite, smectite, hectorite, sodium silicic mica, sodium tainiolite, or lithium tainiolite.

5. A cosmetic comprising the gelling agent according to any one of claims 1 to 4.

6. The cosmetic according to claim 5, further comprising oil in a liquid state at 25°C.

7. The cosmetic according to claim 6, wherein the oil comprises silicone oil.

8. A method for producing a gelling agent, comprising a step of:
treating a water-swellable clay mineral with ammonium group-modified organopolysiloxane represented by the following average composition formula (1),
(R¹₃SiO_{1/2})ₐ₁(R²ₙR¹₃₋ₙSiO_{1/2})ₐ₂(R¹₂SiO_{2/2})_{b}(R¹SiO_{3/2})_{c}(SiO_{4/2})_{d} (1)
in the formula (1), R¹ independently represents a group selected from an alkyl group having 1 to 10 carbon atoms, an aryl group having 6 to 10 carbon atoms, and an arylene group having 7 to 10 carbon atoms,
R² represents an ammonium group represented by the following formula (2),
"a1" satisfies 0 < a1 ≤ 20, "a2" satisfies 0 < a2 ≤ 12, "b" satisfies 0 ≤ b ≤ 150, "c" satisfies 0 ≤ c ≤ 10, and "d" satisfies 0 ≤ d ≤ 5, provided that a1 + a2 + b + c + d is 2 to 170; and "n" represents an integer of 1 to 3, 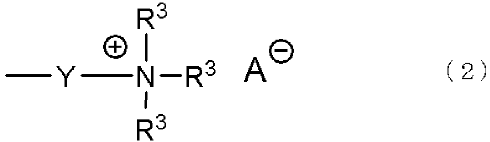
in the formula (2), R³ independently represents a hydrogen atom or a group selected from an alkyl group having 1 to 20 carbon atoms, an aryl group having 6 to 10 carbon atoms, and an arylene group having 7 to 10 carbon atoms,
Y represents a group represented by any of following formulae (3), and
A⁻ represents an anion that is a counterion to an ammonium ion, 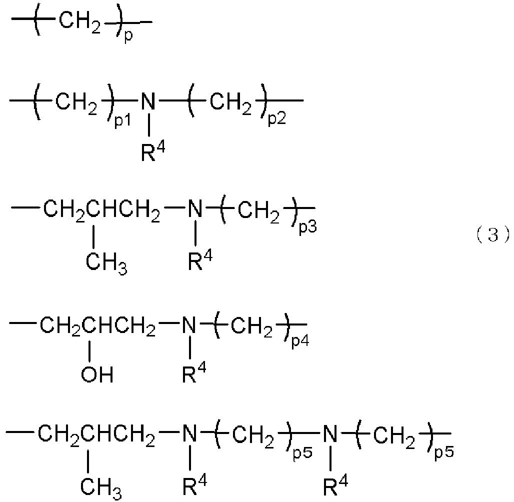
wherein "p" represents an integer of 1≤p≤10; "p1" satisfies 1 ≤ p1 ≤ 10 and "p2" represents an integer of 1 ≤ p2 ≤ 10, provided 1 ≤ p1 + p2 ≤ 15; "p3" represents an integer of 1≤p3≤10; "p4" represents an integer of 1 ≤ p4 ≤ 12; and "p5" represents an integer of 1 ≤ p5 ≤ 10, and
R⁴ represents a hydrogen atom or a group selected from an alkyl group having 1 to 20 carbon atoms, an aryl group having 6 to 10 carbon atoms, and an arylene group having 7 to 10 carbon atoms.

9. The method for producing a gelling agent according to claim 8, wherein the anion represented as A⁻ in the formula (2) is an anion selected from a hydroxide ion, a chloride ion, a bromide ion, a nitrate ion, a hydrogen sulfate ion, a hydrogen carbonate ion, and an acetate ion.

10. The method for producing a gelling agent according to claim 8, wherein a compound represented by the following formula (4) is used as the ammonium group-modified organopolysiloxane, 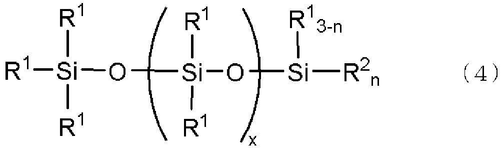 in the formula (4), R¹ ,R², and "n" are same as above; and "x" satisfies 0 ≤ x ≤ 100.

11. The method for producing a gelling agent according to any one of claims 8 to 10, wherein one or more water-swellable clay minerals selected from montmorillonite, saponite, smectite, hectorite, sodium silicic mica, sodium tainiolite, or lithium tainiolite are used as the water-swellable clay mineral.
